# EUROPEAN PATENT APPLICATION

(11) **EP 3 543 692 A1**
(43) Date of publication of application: **25.09.2019**
(21) Application number: 18163328.0
(22) Date of filing: 22.03.2018
(51) Int. Cl.: G01N 33/50

(54) **ADHESION ASSAY**

(71) Applicant: Friedrich-Alexander-Universität Erlangen-Nürnberg, 91054 Erlangen (DE)
(72) Inventor: Zundler, Sebastian, 96049 Bamberg (DE); Neurath, Markus F., 91054 Erlangen (DE); Binder, Marie-Theres, 86169 Augsburg (DE); Becker, Emily Marie, 91056 Erlangen (DE)
(74) Representative: Wünsche, Annelie

(57) **Abstract**

The present invention relates to a method for determining whether a patient with inflammatory bowel disease (IBD) will be responsive to an inhibitor of the interaction of endothelial adhesion molecules with gut homing receptors and kits for utilizing this method. In particular, the invention relates to a method for determining whether a patient with IBD is responsive to vedolizumab. The invention also encompasses a method for determining whether a candidate compound is suitable for the treatment of IBD.

## Description

### Field of the invention

The present invention relates to a method for determining whether a patient with inflammatory bowel disease (IBD) will be responsive to an inhibitor of the interaction of endothelial adhesion molecules with gut homing receptors and kits for utilizing this method. In particular, the invention relates to a method for determining whether a patient with IBD is responsive to vedolizumab. The invention also encompasses a method for determining whether a candidate compound is suitable for the treatment of IBD.

### Background of the invention

Cell trafficking is an essential event for the function of our immune system since it facilitates patrolling of immune cells, antigen presentation and recognition and coordination of infiltration to sites of inflammation. Therefore, it is also implicated in pathological conditions mediated by the immune system including the IBDs Crohn's disease (CD) and ulcerative colitis (UC) as well as unclassified inflammatory bowel disease (IBDU or IBD-U). In particular, IBD is characterized by an intestinal immune cell infiltrate and gut homing (i.e., the extravasation of cells from the blood to the intestinal tissue) of lymphocytes has been recognized as a promising target for therapy. A main mediator of this process is the gut homing integrin α4β7. This integrin is induced in the gut-associated lymphoid tissue in the presence of retinoic acid and binds to the addressin mucosal addressin vascular cell adhesion molecule (MAdCAM)-1 that is almost exclusively expressed on intestinal high endothelial venules. This interaction is functionally important in the process of gut homing, which consists of cell rolling and tethering leading to slow-down and disposition for subsequent cell activation, resulting in affinity modulation of integrins, which may then mediate firm adhesion and, finally, transendothelial migration. Hence, vedolizumab, an anti-α4β7 integrin antibody impeding the adhesion step of gut homing has been successfully evaluated in large clinical trials and has been approved for clinical therapy of both CD and UC. Moreover, several other compounds interfering with cell trafficking in IBD are currently in development, including the anti-β7 antibody etrolizumab, which is thought to additionally interfere with epithelial retention of homed lymphocytes, and the anti-MAdCAM-1 antibody SHP647. Thus, a new family of "anti-adhesion therapies" is emerging.

Despite this large clinical success, appropriate tools to study the adhesion of immune cells to their endothelial addressin counterparts and to evaluate the potential of anti-integrin antibodies to inhibit this process are limited.

Moreover, anti-adhesion therapies are only effective in a part of the IBD patients (Feagean et al. 2013; Sandborn et al. 2013; Vermeire et al., 2014; Vermeire et al., 2017) and it is not possible to predict the success of treatment in individual patients (Zundler et al. 2017). Moreover, anti-adhesion therapies are costly and considered to be "slowly acting", meaning that several months of treatment are necessary to judge about treatment success. Therefore, it is particularly important to provide pre-treatment methods to test whether the inhibitors will show a treatment effect. So far, there are no such methods to predict the clinical response to anti-adhesion therapies, which constitutes an important problem in the treatment of IBD patients. Thus, tools to predict success of these comparably slowly acting therapies inhibiting the interaction of endothelial adhesion molecules with gut homing receptors - like vedolizumab - would be highly desirable.

### Objectives and Summary of the Invention

Hence, a first aspect of the invention relates to a method for determining whether a patient with inflammatory bowel disease will be responsive to an inhibitor of the interaction of endothelial adhesion molecules with gut homing receptors comprising the following steps:
(i) Moving a composition comprising cells of the patient along a surface coated with an endothelial adhesion molecule;
(ii) Detecting the gut homing receptor mediated adhesion of the cells to the surface coated with the endothelial adhesion molecule;
(iii) Predicting whether the patient with IBD will be responsive to the inhibitor of the interaction of endothelial adhesion molecules with gut homing receptors based on the result of step (ii).

In specific embodiments, in step (i), the composition comprising cells is moved through an elongated structure wherein the surface of the inner side of the elongated structure is coated with an endothelial adhesion molecule.

In other words, some embodiments refer to a method for determining whether a patient with inflammatory bowel disease (IBD) will be responsive to an inhibitor of the interaction of endothelial adhesion molecules with gut homing receptors comprising the following steps:
(i) Moving a composition comprising cells of the patient through an elongated structure, wherein the surface of the inner side of the elongated structure is coated with an endothelial adhesion molecule;
(ii) Detecting the gut homing receptor mediated adhesion of the cells to the surface coated with the endothelial adhesion molecule, wherein the surface adhesion of cells treated with the inhibitor is compared to control cells;
(iii) Predicting whether the patient with IBD will be responsive to the inhibitor of the interaction of endothelial adhesion molecules with gut homing receptors based on the result of step (ii).

Preferably, the elongated structure is a capillary.

In some embodiments, in step (ii), the adhesion of untreated cells to the coated surface is measured.

The adhesion of the untreated cells to the coated surface is indicative for a response of the patient with IBD to the inhibitor of the interaction of endothelial adhesion molecules with gut homing receptors. Thus, by measuring the adhesion of untreated cells, it can be easily determined whether a patient could benefit from an anti-adhesion therapy, i.e. from a therapy with an inhibitor of the interaction of endothelial adhesion molecules with gut homing receptors. More specifically, if adhesion of the untreated cells is above a predetermined threshold, this is indicative for a response of the patient with IBD to the inhibitor of the interaction of endothelial adhesion molecules with gut homing receptors.

Alternatively, in step (ii) the surface adhesion of cells treated with the inhibitor is compared to the surface adhesion of control cells. Thereby, the decreased surface adhesion of the treated cells in comparison to the surface adhesion of the control cell is indicative for the efficacy of the inhibitor.

In some embodiments, the gut homing receptor is integrin α4β7.

In preferred embodiments, the endothelial adhesion molecule is an addressin, such as MAdCAM-1 or VCAM-1.

Typically, the cells are leukocytes, such as granulocytes, monocytes or lymphocytes. Preferably, the cells are lymphocytes, such as T cells (e.g., CD4+ T cells or CD 8+ T cells) or B cells.

Preferably, the inhibitor of the interaction of endothelial adhesion molecules with gut homing receptors is an antibody targeting endothelial adhesion molecules or an antibody targeting gut homing receptors. Preferably, the inhibitor of the interaction of endothelial adhesion molecules with gut homing receptors is selected from the group consisting of vedolizumab, natalizumab, etrolizumab and SHP647.

In some embodiments, IBD is selected from the group consisting of CD and UC and IBDU.

In exemplary embodiments, in step (i) the cells are moved through the elongated structure by a peristaltic pump.

In some embodiments, the detection in step (ii) is performed by microscopy, such as fluorescence microscopy or dark-field microscopy. Thus in one embodiment, the detection is fluorescence detection. Accordingly, the cells may be fluorescently labeled.

### Figure Legends

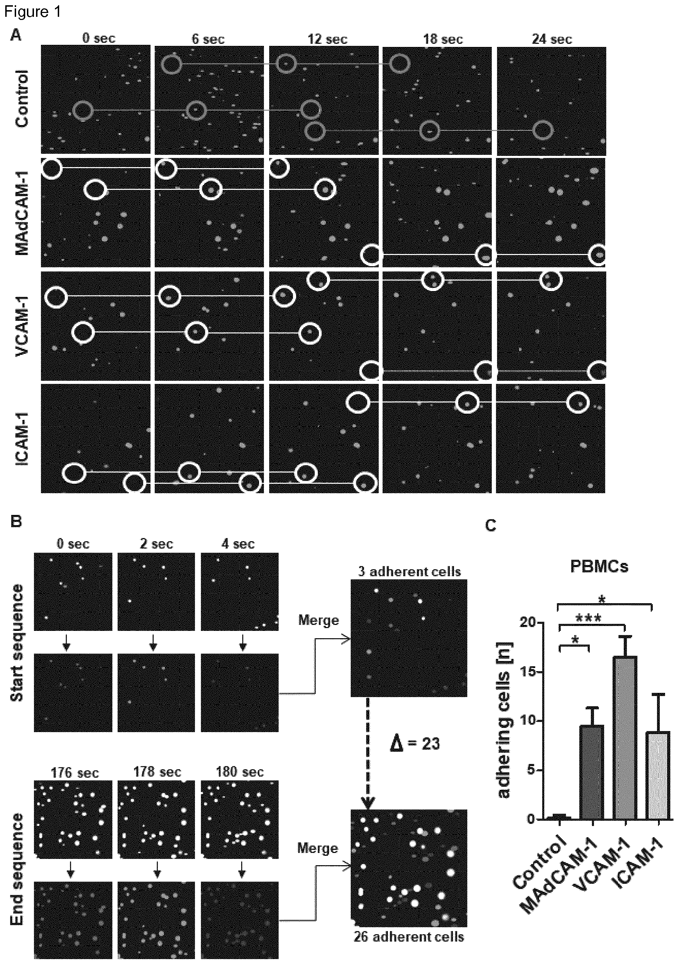

**Figure 1****: Dynamic adhesion assays for real-time investigation of integrin-addressin interaction of human cells from IBD patients**
   (A) Representative time-lapse images from dynamic adhesion assays taken at the indicated time points after start of the experiment. Peripheral blood mononuclear cells (PBMCs) were labeled with carboxyfluorescein succinimidyl ester (CFSE) and perfused through ultra-thin capillaries coated with or without the indicated addressins. CFSE signal is displayed in green. Red circles connected by red lines indicate positions with a cell in the second, but without a cell in the first and third circle, i.e. without dynamic adhesion. White circles connected by white lines indicate positions without a cell in the first circle, but a cell in the second and third circle, i.e. demonstrating a newly adhering cell.
   (B) Description of dynamic adhesion assay quantification. Time-lapse images were taken by confocal microscopy every two seconds for three minutes. The first three ("start sequence") and last three images ("end sequence") were selected and ImageJ was used to color them in red, green and blue. Subsequent overlay of the three images allowed identification of adhering cells, since in positions with the same cell in all three positions red, green and blue merged to white. The difference in white cells in the start and end sequence was determined to quantify the number of adhering cells over three minutes.
   (C) Dynamic adhesion of PBMCs to uncoated control capillaries and capillaries coated with mucosal addressin vascular cell adhesion molecule (MAdCAM)-1, vascular cell adhesion molecule (VCAM)-1 and intercellular cell adhesion molecule (ICAM)-1 (n = 6 per group).

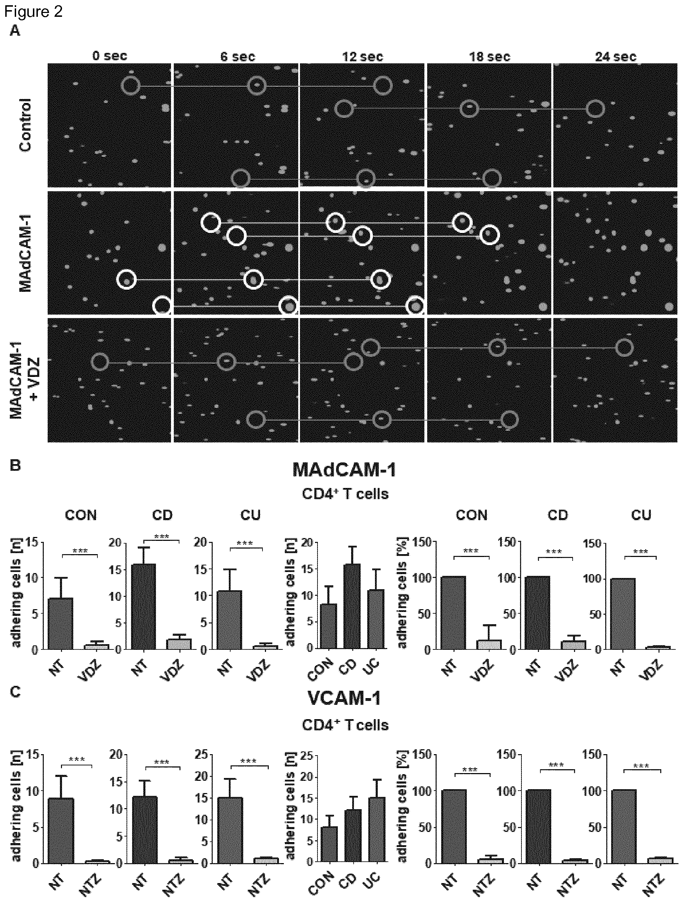

**Figure 2****: Vedolizumab and natalizumab block dynamic adhesion of CD4⁺ T cells from IBD patients to MAdCAM-1 and VCAM-1, respectively**
   (A) Representative time-lapse images from dynamic adhesion assays. CD4⁺ T cells were purified from the peripheral blood of a patient with ulcerative colitis (UC), labeled with CFSE, treated without (NT) or with vedolizumab (VDZ) and perfused through capillaries coated with or without MAdCAM-1. Connected red and white circles depict non-adhering and adhering cells, respectively.
   (B, C) Dynamic adhesion assays with CD4⁺ T cells from control donors (CON), patients with Crohn's disease (CD) and UC in MAdCAM-1- (B) and VCAM-1- (C) coated capillaries as indicated. Left panels: Absolute number of adhering cells after treatment with and without vedolizumab (B) and natalizumab (C). Middle panels: Adhesion of untreated cells in the different groups. Right panels: Relative adhesion of cells after treatment with and without vedolizumab (B) and natalizumab (C) (n = 5-6 per group).

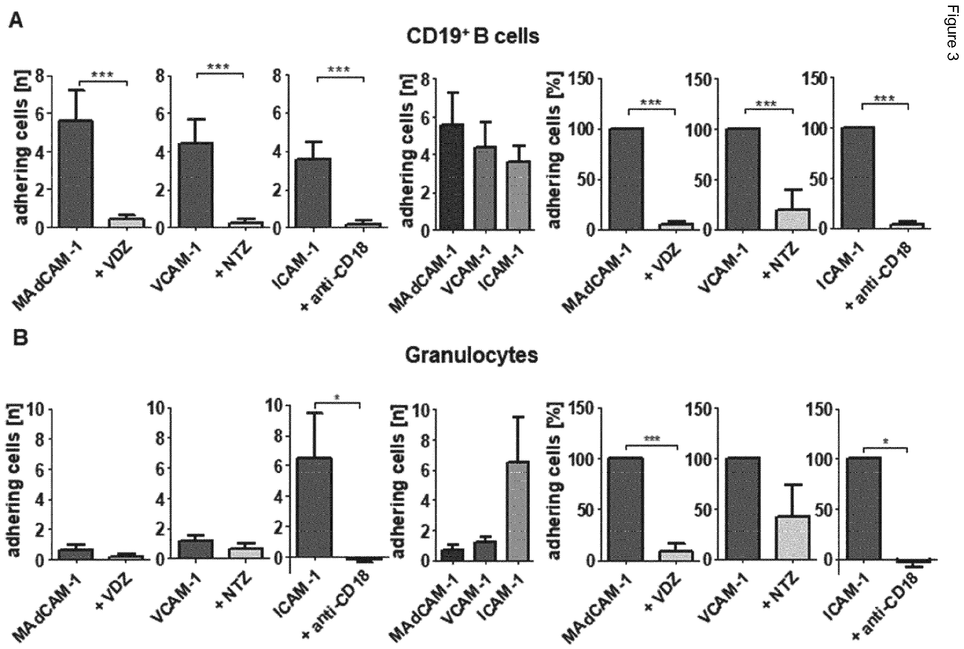

**Figure 3****: Dynamic adhesion of B lymphocytes and granulocytes to gut homing addressins is blocked by specific anti-integrin antibodies**
   Dynamic adhesion assays with CD19⁺ B cells (A) and granulocytes (B) from control donors in capillaries coated with MAdCAM-1, VCAM-1 and ICAM-1 as indicated. Left panels: Absolute numbers of adhering cells after treatment with or without vedolizumab (MAdCAM-1 capillaries), natalizumab (VCAM-1 capillaries) anti-CD18 antibodies (ICAM-1 capillaries). Middle panel: Adhesion of untreated cells to the three addressins. Right panels: Relative adhesion of cells to the indicated addressins after treatment with or without the indicated antibodies (n= 5-6 per group).

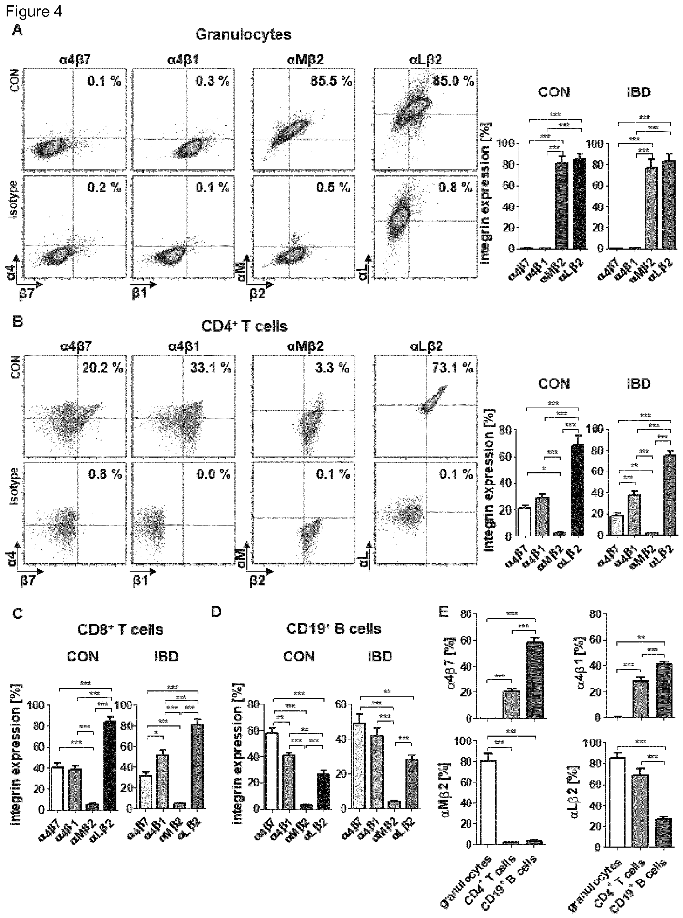

**Figure 4****: Differential expression of integrins on different leukocyte subsets**
   (A, B) Left panels: Upper row: Representative flow cytometric data showing the expression of α4β7, α4β1, αMβ2 and αLβ2 integrins on granulocytes (A) and CD4⁺ T cells (B). Lower row: Representative isotype control stainings with isotypes for β7, β1, αM and β2 integrin (from left to right). Right panels: Flow cytometric expression of α4β7, α4β1, αMβ2 and αLβ2 integrins on granulocytes (A) and CD4⁺ T cells (B) from control donors (n = 5-6) and IBD patients (n = 6-14). (C, D) Flow cytometric expression of α4β7, α4β1, αMβ2 and αLβ2 integrins on CD8⁺ T cells (C) and CD19⁺ B cells from control donors (n = 6) and IBD patients (n = 14). (E) Comparison of flow cytometric expression of α4β7, α4β1, αMβ2 and αLβ2 integrins on granulocytes, CD4⁺ T cells and CD19⁺ B cells in control donors (n = 5-6).

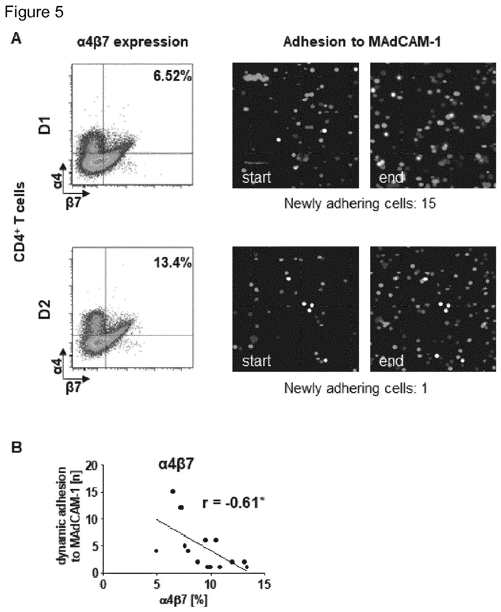

**Figure 5****: Association of integrin expression and dynamic adhesion**
   (A) Representative flow cytometry showing α4β7 integrin expression in CD4⁺ T cells and corresponding start and end sequences from dynamic adhesion assays with CD4⁺ T cells transfused through ultra-thin capillaries coated with MAdCAM-1 from two control donors (D1 and D2).
   (B) Correlation of individual expression of α4β7 on CD4+ T cells with individual adhesion to MAdCAM-1 (n = 15). A regression line and Pearson's r is indicated.

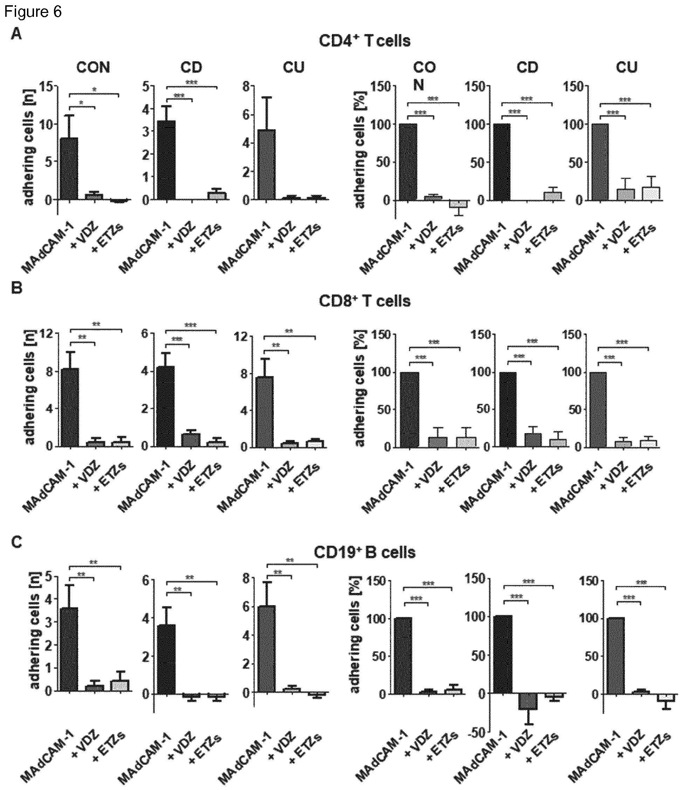

**Figure 6****: Similar inhibition of dynamic adhesion to MAdCAM-1 by vedolizumab and etrolizumab-s**
   CD4⁺ T cells (A), CD8⁺ T cells (B) and CD19+ B cells (C) were treated with or without vedolizumab and etrolizumab-s prior to perfusion through MAdCAM-1-coated capillaries. Left panels: Absolute number of adhering cells. Etrolizumab-s refers to etrolizumab-surrogate, an antibody having the same antigen binding region as etrolizumab but having a rat backbone instead of the humanized backbone. Right panels: Relative adhesion of cells after treatment with and without vedolizumab and etrolizumab-s (n = 5-7 per group).

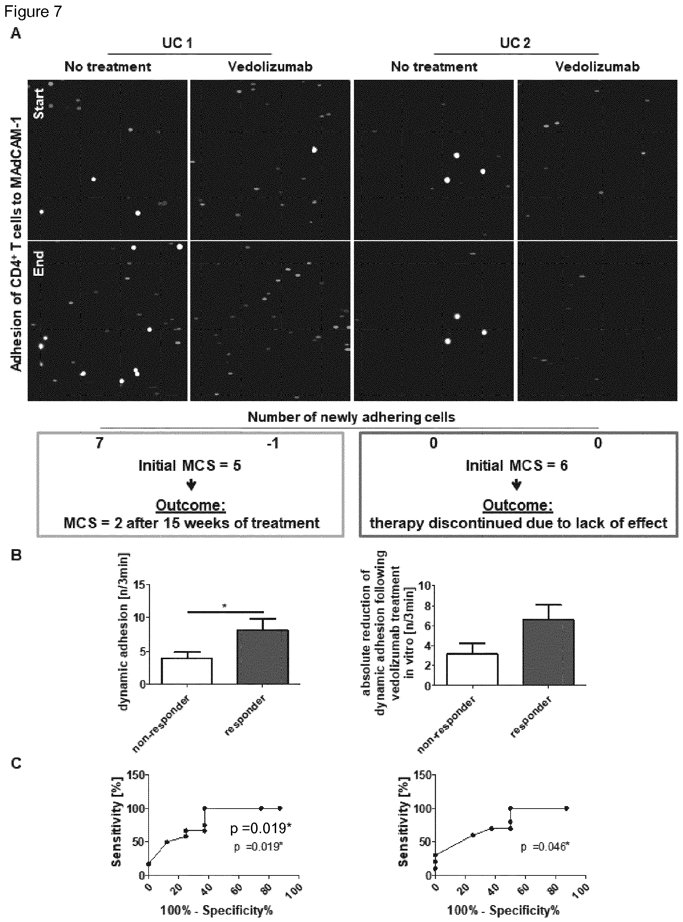

**Figure 7****: Dynamic adhesion assays as a potential tool for prediction of response to vedolizumab therapy**
   (A) CD4⁺ T cells from two UC patients were treated with or without vedolizumab and used in dynamic MAdCAM-1 adhesion assays prior to initiation of clinical treatment with vedolizumab. Start and end sequences are presented to demonstrate dynamic adhesion. Quantification of dynamic adhesion and clinical course under therapy are denoted below.
   (B) Left panel: Patients that respond to the therapy ("responder") show a significant higher adhesion to MAdCAM-1 compared to patients not responding to the therapy ("non-responder"). Right panel: Responders show a higher reduction of the adhesion to MAdCAM-1 after in vitro treatment compared to non-responders.
   (C) Receiver-Operator-Characterisic: Left panel: Dynamic adhesion of untreated cells of more than 8.5 cells per 3 min predicts a treatment response with 100 % sensitivity and a specificity of 62.5 %. Reduction of dynamic adhesion by more than 8.5 cells per 3 min following vedolizumab treatment in vitro predicts a treatment response with 100 % sensitivity and a specificity of 50 %.

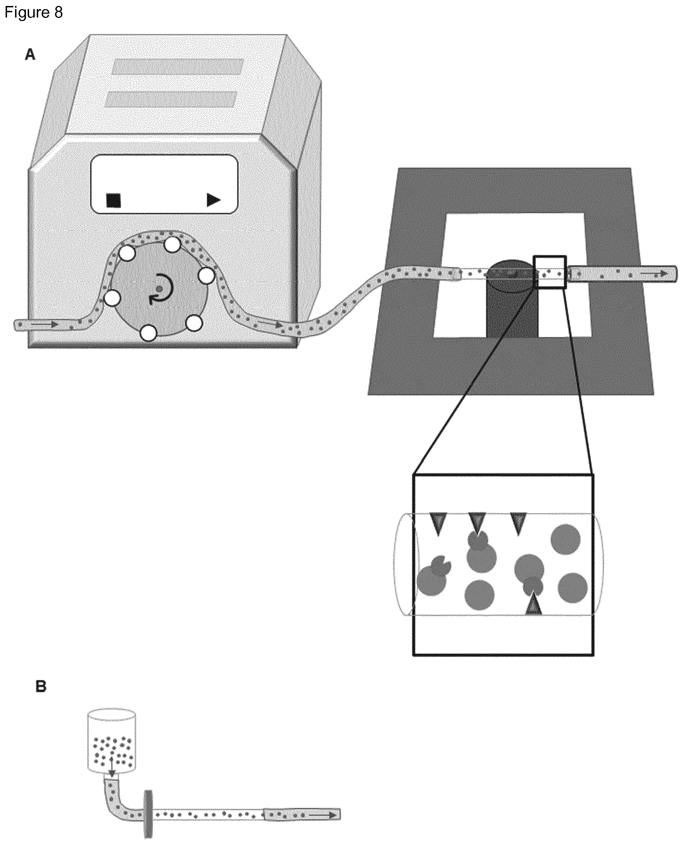

**Figure 8****:Supplementary** **Figure 1****: Technical set-up of dynamic adhesion assays**
   (A)Schematic drawing of dynamic adhesion assay set-up. An ultra-thin capillary coated with an addressin is connected to plastic tubing, which is inserted to a peristaltic pump with adjustable flow rate. Human peripheral blood cells are labeled with CFSE and drawn into the tubing. Confocal time-lapse microscopy is used to record and analyze dynamic adhesion.
   (B) Alternative embodiment in which the flow is produced by gravity-forced perfusion through the capillary wherein the reservoir is placed higher than the capillary. Perfusion may be regulated by a perfusion regulator.

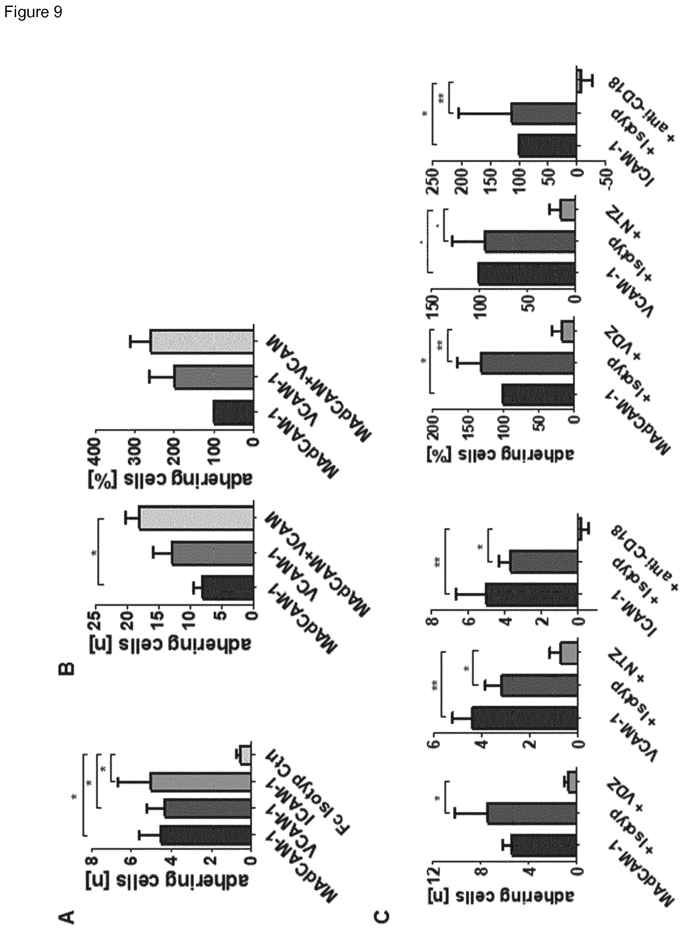

**Figure 9****: Isotype control experiments**
   (A) Dynamic adhesion assays with PBMCs from control donors in capillaries coated with Fc chimera of MAdCAM-1, VCAM-1, ICAM-1 and Fc isotype control. Absolute numbers of adhering cells are indicated (n = 6).
   (B) Dynamic adhesion assays with CD4⁺ T cells from control donors in capillaries coated with MAdCAM-1, VCAM-1 or both. Absolute numbers of adhering cells (left) and numbers relative to MAdCAM-1 (right) are indicated (n = 7).
   (C) Dynamic adhesion assays with PBMCs from control donors treated with vedolizumab (VDZ) or isotype control in capillaries coated with MAdCAM-1, VCAM-1 or ICAM-1 as indicated. Absolute (upper panels) and relative numbers (lower panels) of adhering cells are indicated (n = 5-6 per group).

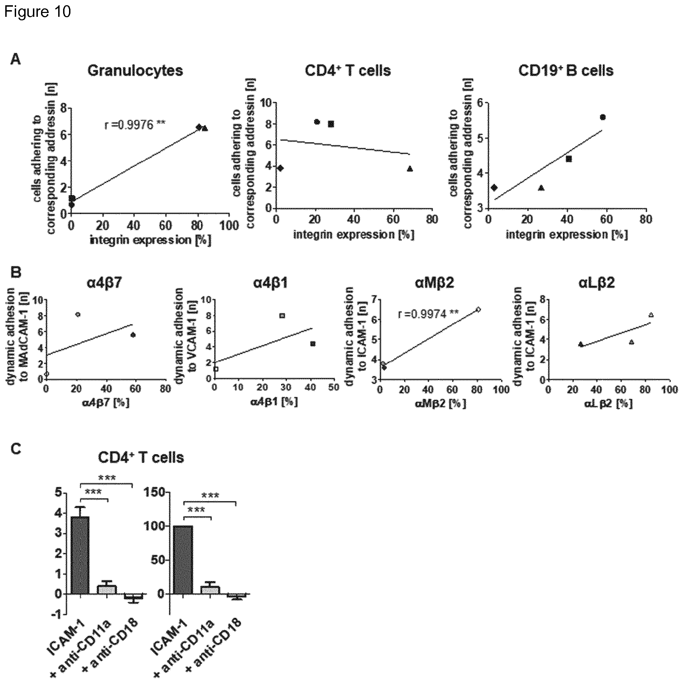

**Figure 10****: Adhesion of CD4⁺ T cells to ICAM-1 and association of integrin expression and dynamic adhesion**
   (A) Correlation of α4β7 (●), α4β1 (■), αMβ2 (◆) and αLβ2 (▲) expression with dynamic adhesion to MAdCAM-1, VCAM-1, ICAM-1 and ICAM-1 in granulocytes, CD4+ T cells and CD19+ B cells as indicated.
   (B) Correlation of granulocyte, CD4⁺ T cell and CD19⁺ B cell adhesion to MAdCAM-1, VCAM-1, ICAM-1 and ICAM-1 with expression of α4β7, α4β1, αMβ2 and αLβ2, respectively (from left to right).
   (C) Dynamic adhesion of CD4⁺ T cells from control donors treated with or without anti-CD11a and anti-CD18 antibodies perfused through ICAM-1-coated capillaries (n = 5).

In (B) and (C) means from flow cytometry shown in Fig. 4 and means from dynamic adhesion assays shown in Fig. 2, 3 and Fig. 10 are correlated. Regression lines are indicated and Pearson's r is noted for significant correlations.

### DETAILED DESCRIPTION OF THE INVENTION

Before the invention is described in detail with respect to some of its preferred embodiments, the following general definitions are provided.

The present invention as illustratively described in the following may suitably be practiced in the absence of any element or elements, limitation or limitations, not specifically disclosed herein.

The present invention will be described with respect to particular embodiments and with reference to certain figures but the invention is not limited thereto but only by the claims.

Where the term "comprising" is used in the present description and claims, it does not exclude other elements. For the purposes of the present invention, the term "consisting of" is considered to be a preferred embodiment of the term "comprising of". If hereinafter a group is defined to comprise at least a certain number of embodiments, this is also to be understood to disclose a group which preferably consists only of these embodiments.

Where an indefinite or definite article is used when referring to a singular noun, e.g. "a", "an" or "the", this includes a plural of that noun unless something else is specifically stated. The term "about" in the context of the present invention denote an interval of accuracy that the person skilled in the art will understand to still ensure the technical effect of the feature in question. The term typically indicates deviation from the indicated numerical value of ±10%, and preferably of ±5%.

Technical terms are used by their common sense. If a specific meaning is conveyed to certain terms, definitions of terms will be given in the following in the context of which the terms are used.

A first aspect of the invention relates to a method for determining whether a patient with IBD will be responsive to an inhibitor of the interaction of endothelial adhesion molecules with gut homing receptors comprising the following steps:
(i) Moving a composition comprising cells of the patient along a surface coated with an endothelial adhesion molecule;
(ii) Detecting the gut homing receptor mediated adhesion of the cells to the surface coated with the endothelial adhesion molecule;
(iii) Predicting whether the patient with IBD will be responsive to the inhibitor of the interaction of endothelial adhesion molecules with gut homing receptors based on the result of step (ii).

"Moving a composition comprising cells along a surface" means that there is a flow produced so that the composition comprising cells is moved along the surface. The surface may be bent to form a gutter.

Preferably, this flow mimics the blood stream through the blood vessels involved in the gut homing process. Thus, in a preferred embodiment, in step (i) the composition comprising cells is moved through an elongated structure wherein the surface of the inner side of the elongated structure is coated with an endothelial adhesion molecule.

Accordingly, some embodiments refer to a method for determining whether a patient with IBD will be responsive to an inhibitor of the interaction of endothelial adhesion molecules with gut homing receptors comprising the following steps:
(i) Moving a composition comprising cells of the patient through an elongated structure, wherein the surface of the inner side of the elongated structure is coated with an endothelial adhesion molecule;
(ii) Detecting the gut homing receptor-mediated adhesion of the cells to the surface coated with the endothelial adhesion molecule;
(iii) Predicting whether the patient with IBD will be responsive to the inhibitor of the interaction of endothelial adhesion molecules with gut homing receptors based on the result of step (ii).

The elongated structure may be a tube, preferably a capillary, for example a miniature borosilicate capillary. The surface, in particular the elongated structure, such as the capillary may contain folds or irregularities which increase the surface.

Typically, the composition comprising cells is perfused through the elongated structure with a speed of 0.00025 µL/(µm^{2*}min) to 0.025 µL/(µm^{2*}min), preferably 0.00075 µL/(µm^{2*}min) to 0.0125 µL/(µm^{2*}min) , more preferably 0.00125 µL/(µm^{2*}min), most preferably 0.0025 µL//(µm^{2*}min).

The movement of the composition might be achieved by connecting the elongated structure, e.g. the capillary, to a pump, e.g. by tubes, such as plastic tubes. The pump may be for example a peristaltic pump. Alternatively, the flow might be generated by gravity, e.g. by positioning a reservoir comprising the composition with the cells on a higher level than the connected capillary. The set-up might further include a perfusion controller.

The concentration of the cells in the composition is about 10³ to 10⁹ cells/mL, preferred 10⁴ to 10⁸ cells/mL, more preferred 0.1*10⁶ to 10⁷ cells/mL, even more preferred 1*10⁶ to 5*10⁶ cells/mL, most preferred 1.5*10⁶ cells/mL.

The composition comprising cells may further contain an adhesion buffer. An exemplary adhesion buffer might have a pH of 7.4, and might contain 150 mM NaCl, 10 mM HEPES, 1 mM CaCl₂, 1 mM MgCl₂, 1mM MnCl₂. The skilled person understands that the adhesion buffer may have also another composition and pH and knows how to prepare such a buffer. The adhesion buffer may contain components that mediate gut homing, for example chemokines.

The endothelial adhesion molecule may be coated on the surface with a concentration of 0.1 µg/mL to 100 µg/mL, preferred 0.5 µg/mL to 50 µg/mL, more preferred 1 µg/mL to 10 µg/mL, such as 5 µg/mL.

The skilled person understands that the term "coated with an endothelial adhesion molecule" means that the surface is coated with at least one endothelial adhesion molecule. In other words, also a combination of two, three or more endothelial adhesion molecules can be applied to the surface. Moreover, the surface could be further coated with additional molecules, such as selectins.

The detection of the gut homing receptor mediated adhesion of the cells in step (ii) might be performed by standard techniques known to the skilled person. In principle, the accumulation of the cells on the surface is measured. This may be carried out by measuring an increasing signal of the labeled cells, by cell counting or by detecting a change in color induced by the accumulation of the cells in a medium capable of switching color dependent on the concentration of the (labeled) cells. Thereby standard microscopy techniques, such as wide field or confocal microscopy, optionally in combination with image analysis tools may be employed. The microscopy may be dark-field microscopy or fluorescence microscopy. In one embodiment, fluorescence detection may be employed on fluorescently labeled cells.

In a specific embodiment, the number of newly adhering cells is detected by time-lapse confocal microscopy in a defined time interval. The time interval may be in the range of seconds to hours, such as 10 s to 1 hour, 30 s to 30 min, 1 min to 10 min, 2 min to 5 min, such as an interval of 3 min.

In a specific embodiment, the number of newly adhering cells is detected by time-lapse confocal microscopy.

In some embodiments, in step (ii) the adhesion of untreated cells to the coated surface is measured.

The adhesion of the untreated cells to the coated surface is indicative for a response of the patient with IBD to the inhibitor of the interaction of endothelial adhesion molecules with gut homing receptors. In particular, if adhesion of untreated cells is above a predetermined threshold, this is indicative for a response of the patient with IBD to the inhibitor of the interaction of endothelial adhesion molecules with gut homing receptors. The skilled person is aware of techniques for determining a threshold for adhesion on the untreated cells, which is indicative for the efficacy of the inhibitor.

For example, the threshold may be more than 5 cells/ 3 minutes, preferably 8.5 cells / 3 minutes or more. This value of cell adhesion may be achieved at a concentration of 1.5 million cells/mL and determined by confocal time-lapse microscopy as described elsewhere herein. The diameter of the capillary may be for example 4000 µm², hence the cells might be perfused through the capillary with a speed of 0.0025 µL/(µm^{2*}min).

The terms "efficacy" or "efficacious" as used herein, mean that the inhibitor of the interaction of endothelial adhesion molecules with gut homing receptors shows a treatment effect, i.e. the cure or amelioration of IBD. Such a treatment effect may depend on the interaction of endothelial adhesion molecules with gut homing receptors which may lead to the cure or amelioration of IBD.

The skilled person understands that a specific threshold indicative for a response of the patient with IBD to the inhibitor of the interaction of endothelial adhesion molecules with gut homing receptors may be determined in a prospective study with an appropriate number of patients treated with the inhibitor assessing the adhesion of untreated cells to the coated surface prior to therapy and subsequent response to the therapy by pre-specified endpoints of clinical, endoscopic and/or histological response and/or remission, such as decrease in clinical or endoscopic disease activity indices. By receiver-operator-characteristic analysis, the value obtained for cell adhesion with best discrimination between responding and non-responding (or remitting and non-remitting) patients can be identified as threshold.

Exemplarily, the threshold may be determined by the following method:
1.) Isolation of CD4+ T cells from the peripheral blood of IBD patients prior to the initiation of the treatment, e.g. less than 4 hours, less than 12 hours, less than 24 hours, less than 7 days, less than 30 days prior to initiation of treatment with vedolizumab and determination of adhesion to MAdCAM-1;
2.) Determination of clinical (e.g., HBI [Harvey-Bradshaw-Index] or CDAI [Crohn's Disease acitivity index] score for CD, PMS [partial Mayo Score] for UC) and/or endoscopical disease activity (e.g. CDEIS [Crohn's disease endoscopic index of severity] for CD, EMS [endoscopic Mayo score] for UC) prior to initiation of treatment;
3.) Determination of clinical and/or endoscopic disease activity after 14 weeks of treatment (or 12-16 weeks, or 6-60 weeks);
4.) Judgment about clinical and/or endoscopic response and/or remission based on difference between activity after 14 weeks of treatment and prior to treatment;
5.) Comparison of CD4+ T cell adhesion to MAdCAM-1 prior to therapy in responders (e.g. ΔHBI ≤ -3 in CD, ΔPMS ≤ -2) vs. non-responders and remitters (e.g. HBI ≤ 4 in CD, PMS ≤ 1 in UC) vs. non-remitters and ROC analysis

The skilled person understands that this method can be adapted, e.g. in step 1.) different cell types (e.g. CD8+ T cells, CD19+ B cells, monocytes, granulocytes), different inhibitors (etrolizumab, natalizumab, SHP647, etc.), and/or different adhesion molecules could be used (VCAM-1, ICAM-1), in step 2.) for example alternative methods such as histologic or sonographic assays could be employed, in steps 3.) and 4.) a different time interval depending on the expected response could be used, in step 5.) different citeria for the determination of response und remission could be applied.

In an alternative embodiment, in step (ii) the surface adhesion of cells treated with the inhibitor is compared to the surface adhesion of control cells. Thereby, the decreased surface adhesion of the treated cells in comparison to the surface adhesion of the control cell is indicative for the efficacy of the inhibitor.

The terms "cells treated with inhibitor" or "treated cells" refer to cells that are either (a) incubated with the inhibitor of interaction of endothelial adhesion molecules with gut homing receptors prior to step (i) or (b) treated with the inhibitor of interaction of endothelial adhesion molecules with gut homing receptors by addition to the adhesion buffer during step (i) and (ii). Regarding (a), the cells may be incubated with the inhibitor for 10 min to 10 hour, 20 min to 5 hours, 30 min to 3 hours, e.g. for 1 hour. The incubation may take place before step (i). Typically, a washing step is performed after incubation and before step (i) of the method. For example, in the case of vedolizumab, the binding of the antibody leads to the internalization of the antigen-antibody-complex (Wyant et al, 2013). Therefore, a washing step might not reduce the inhibition.

The term "control cell" or "control cells" as used herein refers to untreated cells or cells treated with an control molecule, in particular control antibody, that is typically not, or substantially not, inhibiting the interaction of endothelial adhesion molecules with gut homing receptors, such as an isotype control antibody.

The term "isotype control antibody" refers to an antibody having the same isotype as the antibody to be tested but not inhibiting the interaction of endothelial adhesion molecules with gut homing receptors

The term "untreated cells" as used herein, refers to cells that have not been incubated with an inhibitor of interaction of endothelial adhesion molecules with gut homing receptors or a potential inhibitor of interaction of endothelial adhesion molecules with gut homing receptors.

In preferred embodiments, the gut homing receptor may be an integrin heterodimer, such as α4β7, α4β1, αMβ2 (CD11b/CD18) or αLβ2 (CD11a/CD18), or any of the integrin monomers thereof.

In some embodiments, the endothelial adhesion molecule is an addressin. Addressins are known to be the ligands to the homing receptors of lymphocytes. The addressin may be selected from the group consisting of mucosal addressin vascular cell adhesion molecule 1 (MAdCAM-1), intercellular adhesion molecule 1 (ICAM-1) and vascular cell adhesion molecule 1 (VCAM-1). Preferably, the addressin is selected from the group consisting of MAdCAM-1 and VCAM-1. For example, Fc chimera of the recombinant addressins (such as rhMAdCAM-1, rhICAM-1 or rhVCAM-1) could be used for coating.

In some embodiments, the endothelial adhesion molecule may be VCAM-1 and the gut homing receptor may be selected from the group consisting of integrin a4 and integrin α4β1. In other embodiments, the endothelial adhesion molecule may be MAdCAM-1 and the gut homing receptor may be selected from the group consisting of integrin α4β7, integrin β7 and integrin a4. In still other embodiments, the endothelial adhesion molecule may be ICAM-1 and the gut homing receptors may be selected from the group consisting of integrin β2, integrin αL, integrin aM, integrin αLβ2 and integrin αMβ2.

Specific embodiments refer to the following combinations of the endothelial adhesion molecule and gut homing receptors:

**Table 1: exemplary combinations of endothelial adhesion molecules and gut homing receptors**

| **endothelial adhesion molecule** | **gut homing receptor** |
|---|---|
| VCAM-1 | integrin α4 |
| VCAM-1 | integrin α4β1 |
| MAdCAM-1 | integrin β7 |
| MAdCAM-1 | integrin α4 |
| MAdCAM-1 | Integrin α4β7 |
| ICAM-1 | integrin β2 |
| ICAM-1 | integrin αL |
| ICAM-1 | integrin αM |
| ICAM-1 | integrin αLβ2 |
| ICAM-1 | integrin αMβ2 |

In a preferred embodiment, the endothelial adhesion molecule is MAdCAM-1 and the gut homing receptor is integrin α4β7.

The cells used in the assays of the invention are preferably cells capable of gut homing. Typically, the cells are leukocytes. In preferred embodiments, the leukocytes are granulocytes, monocytes or lymphocytes, such as T cells, such as CD4⁺ T cells or CD8⁺ T cells, or B cells, such as CD19⁺ B cells. In more preferred embodiments, the cells are T cells, such as CD4⁺ T cells or CD 8⁺ T cells.

The cells may be isolated by standard techniques known to the skilled person, such as standard density gradient centrifugation, optionally followed by cell sorting, e.g. fluorescent activated cell sorting (FACS) or magnetic activated cell sorting (MACS). An exemplary protocol for cell isolation is described elsewhere herein.

The cells may be labeled, in particular in order to enable their detection. The cells may be for example fluorescently labeled. Exemplary labels include fluorescein, e.g. carboxyfluorescein succinimidyl ester, CellTrace Yellow™, CellTrace FarRed™.

The term "inhibitor" includes small molecules, antibodies and binding fragments thereof, non-antibody protein scaffold proteins, aptameres and nucleotide based molecules. In preferred embodiments, the inhibitor is a small molecule or an antibody. Preferably, the inhibitor is an antibody. In some embodiments, the inhibitor of the interaction of endothelial adhesion molecules with gut homing receptors is an antibody targeting endothelial adhesion molecules or an antibody targeting gut homing receptors, such as an anti-integrin antibody. The anti-integrin antibody may target α4β7, α4β1, αMβ2 (CD11b/CD18), αLβ72 (CD11a/CD18) or may specifically target monomers of the integrin complexes, such as an anti-a4 antibody, an anti-β7 antibody, an anti-CD18 antibody or an anti-CD11b antibody. The antibody targeting the endothelial adhesion molecule may target addressins such as MAdCAM-1, VCAM-1 and ICAM-1. The antibody may also target molecules which modulate the interaction between the endothelial adhesion molecules and the gut homing receptors. For example, the antibody may target cytokines or chemokines which modulate the affinity of integrins.

The inhibitor may target the endothelial adhesion molecule and the gut homing receptor directly or may target the interaction between the endothelial adhesion molecule and the gut homing receptor indirectly, by modulating the affinity of the endothelial adhesion molecule and the gut homing receptor via a different molecule which is neither the endothelial adhesion molecule nor the gut homing receptor. An example for an indirect interference is the targeting of cytokines, which influence the cell activation and thereby modulate the affinity of the integrins, such as vercirnon, inhibiting CCR9 and edelumab targeting IP-10/CXCL10.

In some embodiments, the inhibitor of the interaction of endothelial adhesion molecules with gut homing receptors is selected from the group consisting of vedolizumab (Feagan et al., 2013; Sandborn et al., 2013), natalizumab (Targan et al., 2007), etrolizumab (Vermeire et al., 2014) ,SHP647 (also termed PF-00547659; Vermeire et al. 2017), AJM300 (Yoshimura et al. 2015) , AMG181 (also termed abrilumab; Pan et al. 2014), vercirnon (Feagan et al. 2015) and BMS-936557 (Mayer et al. 2014). Preferably, the inhibitor of the interaction of endothelial adhesion molecules with gut homing receptors is selected from the group consisting of vedolizumab, natalizumab, etrolizumab and SHP647.

AJM300 is a small molecule inhibiting the integrin a4. AMG181, also termed abrilumab is an anti-α4β7 antibody. Vercirnon is a chemokine inhibitor inhibiting CCR9. BMS-936557 is an anti-IP-10/CXC10 antibody.

The skilled person understands that in methods for determining whether a patient with inflammatory bowel disease will be responsive to an inhibitor of the interaction of endothelial adhesion molecules with gut homing receptors, the experimental setup typically contains a molecule that is targeted by the inhibitor. That means that if the assayed inhibitor targets a specific addressin, e.g. MAdCAM-1, the surface may be coated with the respective addressin, e.g. MAdCAM-1. Alternatively, if the assayed inhibitor targets a gut homing receptor, the composition comprising cells may contain cells that express the respective gut homing receptor, e.g. T cells. Moreover, the cell type may express the gut homing receptor that binds to the endothelial adhesion molecules coated on the surface.

In specific embodiments, IBD is selected from Crohn's disease (CD), ulcerative colitis (UC) and unclassified inflammatory bowel disease (IBDU).

The invention further relates to a method for determining whether a candidate compound is suitable for the treatment of IBD comprising the following steps:
(i) Moving a composition comprising cells along a surface coated with an endothelial adhesion molecule;
(ii) Detecting gut homing receptor-mediated adhesion of the cells to the surface coated with the endothelial adhesion molecule, wherein the surface adhesion of cells treated with the inhibitor is compared to control cells;
(iii) Predicting whether a candidate compound is suitable for the treatment of IBD based on the result of step (ii).

In other words, this method allows determining whether the candidate compound is inhibiting the interaction of endothelial adhesion molecules with gut homing receptors.

For the method for determining whether a candidate compound is suitable for the treatment of IBD, the cells of a patient or the cells of a healthy individual can be used.

The term "candidate compound" includes small molecules, antibodies and binding fragments thereof, non-antibody protein scaffold proteins, aptameres and nucleotide based molecules. In preferred embodiments the candidate compound is a small molecule or an antibody. Preferably, the candidate compound is an antibody. In some embodiments, the candidate compound of the interaction of endothelial adhesion molecules with gut homing receptors is an antibody targeting endothelial adhesion molecules or an antibody targeting gut homing receptors, such as an anti-integrin antibody. The anti-intergrin antibody may target α4β7, α4β1, αMβ2 (CD11b/CD18), αLβ72 (CD11a/CD18) or may specifically target monomers of the integrin complexes, such as an anti-CD11b antibody, an anti-CD18 antibody, an anti-α4 antibody or an anti-β7-antibody. The antibody, targeting the endothelial adhesion molecule may target addressins such as MAdCAM-1, VCAM-1 and ICAM-1. The antibody may also target molecules which modulate the interaction between the endothelial adhesion molecules and the gut homing receptors, for example the antibody may target cytokines or chemokines which modulate the affinity of integrins.

The invention further relates to a kit comprising:
- an elongated structure coated at its inner surface with an endothelial adhesion molecule,
- instructions for use,
- optionally an adhesion buffer,
- optionally a labeling compound.

The labeling compound may be suitable for labeling cells as described herein so that the labeled cells can be employed in the detection step (ii) as described herein.

The invention may also encompass a device comprising:
- means for accommodating a capillary,
- means capable of moving the composition comprising cells through the capillary,
- optionally a reservoir capable of accommodating a composition comprising cells,
- optionally a reservoir for incubation of a composition comprising cells with the inhibitor of the interaction of a gut homing receptor with an endothelial adhesion molecule
- optionally a detector capable of detecting the surface adhesion of the cells in the capillary,
- optionally means for determining the number of adhering cells directly (e.g. by identification of adhering cells) or indirectly (e.g. via measurement of fluorescence, brightness, shading or color change),
- optionally means for comparing the determined value with a specific threshold value and/or for calculating the probability of response based on the determined value,
- optionally a display indicating the result of the measurement, the comparison and/or the calculation,
- optionally means for connecting a coated capillary with the reservoir.

The means for accommodating a capillary are for example provided by a capillary accommodator configured to accommodate the capillary. The means for determining the number of adhering cells are for example provided by a cell counter configured to determine the number of adhering cells directly or indirectly. The means for comparing the determined value with a specific threshold are for example provided by a module that is configured to compare the determined value with a specific threshold. The means for calculating the probability of response based on the determined value are for example provided by a module that is configured to calculate the probability of response based on the determined value.

### Experiments

### Materials and Methods:

### Patients with IBD

Peripheral blood for this study was donated by patients with UC (n= 32) and CD (n= 28) at the Outpatient Department the Medical Clinic 1 of the University Hospital Erlangen following written informed consent. The procedure was approved by the Ethics Committee of the Friedrich-Alexander-University Erlangen-Nuremberg. Control blood was obtained from healthy donors (n = 30). Table 1 summarizes the donors' clinical data.

### Cell Isolation, labeling and in vitro treatment

Standard density gradient centrifugation with Pancoll (Pan Biotech) was performed to obtain peripheral blood mononuclear cells (PBMCs) from full blood samples. Where indicated, PBMCs were further purified to CD4⁺ or CD8⁺ T cells or CD19⁺ B cells by using CD4, CD8 or CD19 microbeads (Miltenyi Biotec), respectively, in accordance with the manufacturer's instructions. .Alternatively FACS could be used for purification of cells from PBMCs. For granulocyte isolation the bottommost layer of density gradient centrifugation containing granulocytes and erythrocytes was resuspended in 1% Dextran 500 (Roth) and the erythrocytes left to sediment passively through gravity for 30 minutes (Leppkes et al. 2016). After a washing step, hypotonic lysis was performed to remove the remaining erythrocytes from the granulocyte-rich supernatant. Low-binding tubes were used for all incubation steps to prevent granulocyte adhesion to the surface. For use in microscopy, carboxyfluorescein succinimidyl ester (CFSE) (Life Technologies, Carlsbad, CA) was applied to label the respective cells. Treatment with anti-integrin antibodies was performed in RPMI 1640 medium (Thermo Fisher) with 1% penicillin/streptomycin (Biochrom, Berlin, Germany) and 10% FCS (Pan Biotech) at a concentration of 1.5 million cells/mL for one hour. Natalizumab (Biogen), Vedolizumab (Takeda), the etrolizumab surrogate antibody FIB504 (Genentech), anti-CD11a (HI111, Biolegend) and anti-CD18 antibodies (TS1/18, BioLegend) were used at a concentration of 10 µg/mL.

### Dynamic in vitro adhesion assay

Fc chimera of either rhMAdCAM-1, rhICAM-1(both from R&D Systems) or rhVCAM-1 (BioLegend) were used at a concentration of 5 µg/mL in 150 mM NaCl with 10 mM HEPES to coat the inside of miniature borosilicate capillaries (Vitrocom) for one hour at 37°C. In selected experiments rhMAdCAM-1 and rhVCAM-1 were simultaneously coated to the same capillaries. After careful removal of the coating solution, 5 % BSA in PBS was applied for one hour at 37°C to block unspecific binding sites. Capillaries were connected to plastic tubing that was inserted in a peristaltic pump (Baoding Shenchen Precision Pump Company) with adjustable flow rate. Cells isolated, labeled and treated as detailed above were resuspended in adhesion buffer (pH 7,4; 150 mM NaCl, 10 mM HEPES, 1 mM CaCl₂, 1 mM MgCl₂, 1 mM MnCl₂; Tidswell et al. 1997) at a concentration of 1.5 million cells/mL and perfused through the capillaries at a speed of 10 µL/min.

Time-lapse confocal microscopy (Leica SP8) was used for analyses and clips of three minutes length with pictures taken every two seconds were recorded for every capillary. ImageJ (NIH) software was used for quantification. More precisely, the three initial and the three final images of the recorded clips were merged after previous coloring in blue, red and green. For stationary cells with the same position in all three source images this resulted in white overlay, while moving cells with different positions in the three images maintained the original color assigned in the three source images. The number of newly adhering cells during these three minutes was quantified by counting the number of white cells in the "start sequence" (first three images) and "end sequence" (last three images).

Representative clips were exported to video format for visualization of dynamic adhesion.

### Flow cytometry

PBMCs and granulocytes were isolated as mentioned above and stained with antibodies against CD3 (SK7, Pacific Blue, Biolegend), CD4 (A161A1, APC/Cy7, Biolegend), CD4 (VIT4, FITC, Miltenyi), CD8a (RPA-T8, BV605, Biolegend), CD14 (HCD14, PE, Biolegend), CD16 (3G8, APC/Cy7, Biolegend) CD19 (LT19, FITC, Miltenyi), α4 integrin (MZ18-24A9, Vioblue, Miltenyi Biotec), αM integrin (M1/70, APC, Miltenyi; M1/70, BV785; ICRF, FITC), β7 integrin (FIB27, PE and PerCP/Cy5.5), β1 integrin (TS2/16, AF647), αL integrin (HI111, PE/Cy7) and β2 integrin (TS1/18, PerCP/Cy5.5, all from Biolegend) as well as appropriate isotype control antibodies. In some experiments, cells were fixed with the Foxp3/Transcription Factor Staining Buffer Set (eBioscience). Flow cytometry was performed on an LSR Fortessa instrument.

### Statistics

Statistical comparisons were performed using Graph Pad Prism software (Graph Pad Software) applying ANOVA with Tukey's post-hoc test or student's t test, where applicable. Levels of significance are indicated by asterisks (* p < 0.05, ** p < 0.01, *** p < 0.001). Bar charts display mean values with SEM.

### Results

### Dynamic adhesion assays for real-time investigation of integrin-addressin interaction of human cells from IBD patients

Convenient techniques to study, visualize and quantify the integrin-mediated adhesion of immune cells from IBD patients to addressins like MAdCAM-1, VCAM-1 and ICAM-1 implicated in leukocyte recruitment to the inflamed gut under flow conditions are so far limited. Thus, until now, these interactions have been predominantly explored under static conditions (Tidswell et al.).

We therefore developed a new type of dynamic adhesion assay for the investigation of human integrin-addressin interaction. In this assay, we used ultra-fine glass capillaries and coated the above mentioned addressins to their inside. Leukocytes from human donors were isolated, fluorescently labeled and perfused through the capillaries by a peristaltic pump (*Figure 8*)*.* Thus, our model simulated blood flow through vessels.

Time-lapse confocal microscopy permitted visualization of cells flowing through the capillaries and adhering to coated addressins. In a first series of experiments, PBMCs were obtained from the blood of healthy donors and perfused through capillaries coated with and without MAdCAM-1, VCAM-1 and ICAM-1. While virtually no cells adhered to uncoated capillaries, there was obvious adhesion to capillaries coated with either of the addressins (*Fig. 1A**;*)*.*

To quantify the amount of adhering cells, we identified cells adhering to the capillary by overlay of sequences of three images at the beginning of the experiments and of three images after three minutes as detailed in the methods section providing the opportunity to determine the number of cells newly adhering in the course of this period (*Fig. 1B*). Using this method, significant differences in the adhesion of PMBCs to uncoated vs. coated capillaries could be demonstrated with no significant differences in PBMC adhesion to MAdCAM-1, ICAM-1 and VCAM-1 (*Fig 1C*). To exclude that the observed adhesion was a consequence of unspecific adhesion to the coated proteins and not specifically related to the integrin-addressin interaction, we performed additional experiments, in which we coated isotype IgG-Fc to the inside of the capillaries and perfused them with labeled PBMCs. Again, marked and significant differences between control and addressin-coated capillaries could be observed (*Fig. 9*) confirming that our model is suitable for specific assessment of integrin-addressin interaction.

We had earlier reported that a proportion of α4β7-expressing CD4⁺ T cells co-express α4β1¹². We therefore wondered, what this means for dynamic adhesion in the presence of both MAdCAM-1 and VCAM-1. To address this question, we coated capillaries with either MAdCAM-1, VCAM-1 or both and perfused them with CD4+ T cells. As expected, more cells bound to double-coated capillaries. However, their number was less than the sum of cells bound to MAdCAM-1-coated and VCAM-1-coated capillaries (*Fig. 9*). This is in line with the reported co-expression of integrins since all α4β7-expressing cells may bind to MAdCAM-1 and all α4β1-expressing cells may bind to VCAM-1 but only single-positive cells may additionally adhere, when both addressins are present.

### Vedolizumab blocks dynamic adhesion of CD4⁺ T cells from IBD patients to MAdCAM-1

We purified CD4⁺ T cells from the peripheral blood of IBD patients and control donors and incubated them with or without vedolizumab in vitro prior to perfusion through MAdCAM-1-coated capillaries. While no adhesion occurred in uncoated capillaries, marked adhesion was observed in MAdCAM-1-coated capillaries perfused with untreated cells. To the contrary, only very little adhesion of vedolizumab-treated cells to MAdCAM-1 could be seen (*Fig. 9A*)*.* This could be consistently demonstrated for patients with CD and UC as well as healthy donors with the absolute number of cells adhering to MAdCAM-1 tending to higher levels in IBD patients vs. control donors (*Fig. 2B*)*.*

In an additional round of experiments we coated capillaries with VCAM-1 and treated CD4+ T cells from IBD patients and healthy donors with the anti-α4 antibody natalizumab interfering with binding of α4β1 integrin to VCAM-1 in addition to blocking α4β7-MAdCAM-1 interaction. Similarly, a distinct and significant reduction of T cell adhesion to VCAM-1 after natalizumab treatment compared with no treatment was observed (*Fig. 2C*).

Furthermore, we sought to exclude that the virtual absence of adhesion after treatment with vedolizumab and natalizumab arose from unspecific mechanical prohibition of cell-addressin contact and not from specific disruption of integrin-addressin interaction. Thus, we performed experiments, in which cells were incubated with vedolizumab, natalizumab or unspecific human IgG. As expected, dynamic adhesion after vedolizumab or natalizumab treatment was barely present, while IgG-treated cells showed no impairment in dynamic adhesion compared to untreated cells (*Fig. 9C*)*.* This was consistent with the notion that vedolizumab and natalizumab specifically impede dynamic adhesion to MAdCAM-1 and VCAM-1, respectively, in our model.

### Dynamic adhesion of B lymphocytes and granulocytes to gut homing addressins is blocked by specific anti-integrin antibodies

Subsequently, we raised the question, whether it is also possible to analyze the interaction of integrins expressed on other immune cells with gut homing addressins in our dynamic adhesion assay. Accordingly, CD19⁺ B cells were isolated from the peripheral blood of healthy donors and treated in vitro with or without vedolizumab, natalizumab or anti-CD18 antibodies blocking β2 integrins interacting with ICAM-1. Next, these cells were perfused through capillaries coated with MAdCAM-1, VCAM-1 or ICAM-1, respectively. Indeed, dynamic adhesion to all three addressins occurred with untreated cells, while antibody treatment resulted in a significant reduction (*Fig. 3A*).

Moreover, we isolated granulocytes from the sediment of blood submitted to density gradient centrifugation as stated in the Methods section and used them in analogous assays. Considerable dynamic adhesion to ICAM-1 could be observed and was completely blocked by pretreatment with anti-CD18, but not by unspecific IgG isotype control. To the contrary, adhesion to MAdCAM-1 and VCAM-1 was clearly lower. However, even these low levels further declined after antibody treatment with vedolizumab and natalizumab, respectively (*Fig. 3B**;* *Fig. 9B*).

### CD4+ T cell adhesion to MAdCAM-1 is negatively correlated with α4β7 expression

In view of these results, we wanted to better understand how and to what extent dynamic adhesion to the addressins MAdCAM-1, VCAM-1 and ICAM-1 is related to the expression of the interacting integrins α4β7, α4β1, αMβ2 (CD11b/CD18) and αLβ2 (CD11a/CD18), respectively, in the different leukocyte subsets. To address this question, we stained PMBCs and granulocytes obtained from healthy donors and IBD patients with antibodies for the above mentioned integrins and analyzed their expression by flow cytometry. Large differences could be observed in both the expression of different integrins in a specific cell population and the expression of a specific integrin in different cell populations, whereas these patterns were largely similar in IBD patients and healthy controls.

Expression of α4β7 and α4β1 integrins in granulocytes was virtually absent, while αMβ2 and αLβ2 integrins were expressed on around 80% of the cells (*Fig. 4A*). To the contrary, around 20 % and 30 % of the CD4⁺ T cells expressed α4β7 and α4β1, respectively, but showed almost no expression of αMβ2 and high levels of αLβ2 *(**Fig. 4B*). This pattern was similar in CD8⁺ T cells, although the levels for α4β7 and α4β1 were higher (*Fig. 4C*). Finally roughly half of the CD19⁺ B cells expressed α4β7 on their surface, while α4β1 expression was around 40 % and αLβ2 expression around 25 % (*Fig. 4D*)*.* When comparing the expression of these integrins between different cell populations, it turned out, that expression of both α4β7 and α4β1 is highest in B lymphocytes compared with CD4⁺ T cells and granulocytes, the latter barely expressing either of the dimers. Moreover, αMβ2 integrin is found on the majority of granulocytes but hardly expressed in lymphocytes, while αLβ2 integrin is similarly found on most granulocytes, but also expressed on CD4⁺ T cells and CD19⁺ B lymphocytes to considerable extent (*Fig. 4E*).

These differential expressions, together with the differences observed in adhesion above suggested a certain level of association between expression and function. Since α4β7 and α4β1 were barely expressed in granulocytes and no relevant adhesion to MAdCAM-1 and VCAM-1 could be observed with these cells, but expression of αMβ2 and αLβ2 and the extent of adhesion to ICAM-1 was high, correlation of the mean expression of α4β7, α4β1, αMβ2 and αLβ2 with the mean level of dynamic adhesion to the corresponding addressins showed a clear and highly significant relationship of integrin expression and function in this cell population (*Fig.* 10). A similar trend could be observed in CD19⁺ B cells suggesting that expression of a specific integrin indeed favors adhesion to the respective addressin. However, no such association could be observed in CD4⁺ T cells, thus underscoring that additional cell-specific modulators of integrin-dependent adhesion to addressins exist.

We also analyzed the correlation of integrin expression and function in different cell populations with focus on the specific integrins (*Fig. 10*)*.* Due to low expression of αMβ2 in CD4⁺ T cells and CD19⁺ B cells, but high expression in granulocytes, and corresponding low and high levels of dynamic adhesion to ICAM-1, respectively, a significant association was found for this integrin. In trend, this was also the case for αLβ2, but not for α4β7 and α4β1. This further supported the notion that additional regulatory elements are involved in dynamic adhesion and be especially so for integrins like α4β1 and α4β7 predominantly used by the adaptive immune system, whereas adhesion mediated by the integrins αMβ2 and αLβ2, which are considered more important for innate cells, appears to be a more direct function of expression levels.

Beyond the main cell populations analyzed so far, a multitude of subsets exist that differ in their integrin expression^{6,10} potentially leading to differential dynamic adhesion. Here, we investigated differential integrin expression and function in naive CD4⁺CD45RA⁺ vs. memory/effector CD4⁺CD45RA⁻ T cells. While the expression of α4β7 integrin on naive CD4⁺ T cells was non-significantly lower and dimmer than on memory/effector CD4⁺ T cells, dynamic adhesion of naive CD4⁺ T cells to MAdCAM-1 was increased compared with memory/effector CD4⁺ T cells. This might be explained by an additional role of L-Selectin^{1,2}, a main marker of naive T cells, for interaction with MAdCAM-1¹⁹ and, once more, demonstrates that adhesion to addressins is not a simple function of integrin expression.

Further, we analyzed the association of the individual expression of α4β7 and the dynamic adhesion to MAdCAM-1. Strikingly, we found a statistically significant negative correlation *(**Fig. 5*) suggesting that low expression of α4β7 is associated with increased individual adhesion to MAdCAM-1. This might be due to a particularly important role of the α4β7-MAdCAM-1 pathway in these individuals leading to α4β7-dependent gut homing with subsequent reduction of α4β7-bearing CD4⁺ T cells in the peripheral blood. However, the correlation coefficient of -0.61 suggested that α4β7-dependent adhesion to intestinal vessels is not exclusively related to α4β7 expression, but controlled by additional parameters.

### Similar inhibition of dynamic adhesion to MAdCAM-1 by vedolizumab and etrolizumab-s

In order to use the dynamic adhesion assay for the investigation of aspects with high translational relevance in the context of anti-adhesion therapies, we continued our study with a series of experiments exploring the relative impact of vedolizumab and etrolizumab on inhibiting the adhesion to MAdCAM-1. Etrolizumab is an anti-37 antibody blocking both α4β7 and αEβ7, which is currently clinically tested in a large phase III trial program. Although it is also interfering with α4β7-dependent adhesion to MAdCAM-1, it is so far not clear whether vedolizumab and etrolizumab are similarly efficacious in this regard. Earlier studies with static adhesion assays had shown no difference regarding the adhesion of CD4⁺ and CD8⁺ T cells and, here, we also found no difference in static adhesion with CD19⁺ B cells. Since etrolizumab is not readily available, we used the etrolizumab surrogate antibody FIB504 (etrolizumab-s) in our study, which is composed of an identical antigen recognition site but a murine backbone compared to the humanized antibody.

To investigate dynamic adhesion with these antibodies, we treated CD4⁺ T cells from IBD patients and healthy donors with and without vedolizumab and etrolizumab-s and studied dynamic adhesion to MAdCAM-1-coated capillaries. Adhesion was similarly reduced by both antibodies (*Fig. 6A*) in CD and UC patients as well as healthy donors. Since CD8⁺ T cells have recently been proposed as another important target of anti-adhesion therapies, we performed analogous assays with CD8⁺ T cells. Again, both compounds significantly reduced adhesion, but did not differ in their impact in any of the groups *(**Fig. 6B*). Given the considerable expression of α4β7 on CD19⁺ B cells, we also conducted these assays with peripheral blood B cells. As with T cells, no difference in the effect of vedolizumab and etrolizumab-s on adhesion to MAdCAM-1 was noted (*Fig. 6C*)*.* Taken together, these data suggested that therapy with vedolizumab and potential future therapy with etrolizumab might similarly inhibit dynamic adhesion of lymphocytes from IBD patients to MAdCAM-1 in intestinal vessels.

### Dynamic adhesion assays as a potential tool for prediction of response to vedolizumab therapy

Consistently, in a pilot experiment, we determined the dynamic adhesion of CD4⁺ T cells from two UC patients scheduled to undergo vedolizumab treatment prior to the first treatment. Cells were treated in vitro with or without vedolizumab and dynamic adhesion was quantified. Subsequently, we followed the patients' clinical course. One patient responded to therapy indicated by a drop of the Mayo clinical score from 5 to 2 points. One patient did not respond to therapy and vedolizumab was discontinued (Fig. 7A). Strikingly, a high level of dynamic adhesion of CD4⁺ T cells to MAdCAM-1 had been observed in the former patient before treatment, while not a single cell of the latter patient had adhered to MAdCAM-1. Despite their very preliminary nature, these data are consistent with the notion that pre-treatment assessment of integrin function might potentially be useful to predict the response to anti-adhesion therapies.

To further investigate this possibility, we determined the dynamic adhesion of untreated and vedolizumab-treated CD4⁺ T cells to MAdCAM-1 in a cohort of 18 IBD patients prior to initiation of clinical vedolizumab therapy. Response was prospectively assessed based on clinical, endoscopic and histological parameters. Adhesion of untreated cells to MAdCAM-1 was higher in patients with a response than in patients without a response as was the reduction of adhesion following vedolizumab treatment (Fig. 7B). A receiver-operator-characteristic (ROC) was calculated for both parameters to analyze their suitability for prediction of therapeutic success of vedolizumab. For untreated cells, adhesion of more than 8.5 cells/3 min was best in discriminating between responding and non-responding patients. This threshold had 100 % sensitivity and 62.5 % specificity. The AUC for this ROC analysis was 0.82 (95 % CI 0.62-1.0) with p = 0.019. For treated cells in comparison to untreated cells, a reduction of adhesion of more than 8.5 cells/3 min was best in in discriminating between responding and non-responding patients. This threshold had 100 % sensitivity and 50 % specificity. The AUC for this ROC analysis was 0.78 (95 % CI 0.56-0.99) with p = 0.46. Taken together, these data from a small, but prospective cohort of patients showed that pre-treatment evaluation of dynamic adhesion of untreated or treated cells might indeed be suitable to predict response to treatment with vedolizumab.

### Discussion

Here, we present a method to study the dynamic adhesion of human IBD peripheral blood cells to gut homing integrins in real time, also providing the opportunity to evaluate the effects of anti-adhesion compounds in a smart functional in vitro set-up closely reflecting several aspects of dynamic adhesion to endothelial addressins in intestinal vessels. For the first time, we present data visualizing the disruption of α4β7-mediated adhesion to MAdCAM-1 by vedolizumab and, moreover, demonstrate that the presented dynamic adhesion model is useful to study integrin-addressin interaction and pharmacological interference in a multitude of cells, with a variety of integrins and additional anti-integrin antibodies. Our data suggest that vedolizumab and the investigational agent etrolizumab do not differ in their efficacy to block lymphocyte adhesion to MAdCAM-1 and raise hopes that assessment of dynamic adhesion could be used for predictive analyses with anti-adhesion antibodies.

It seems possible to further optimize the system by coating additional molecules or adding signaling peptides to the adhesion buffer.

We show that vedolizumab treatment of CD4⁺ T cells from patients with UC and CD as well as control donors dramatically reduces dynamic adhesion to MAdCAM-1 confirming current mechanistic concepts on the action of this drug that start from the premise that vedolizumab specifically inhibits binding of α4β7-expressing T lymphocytes to MAdCAM-1 in high endothelial venules of the gut leading to subsequent reduction of inflammatory infiltration to the tissue.

An important and clinically relevant question in this context is whether current anti-adhesion therapy with vedolizumab and future therapies with etrolizumab or anti-MAdCAM-1 could differ in their potential to inhibit α4β7-MAdCAM-1 interactions since this might also affect clinical efficacy. Since clinical head-to-head-trials are so far missing, we considered it important to investigate this point with our dynamic adhesion assays and compared the impact of vedolizumab and etrolizumab-s on dynamic adhesion to MAdCAM-1. Testing CD4⁺ T cells, CD8⁺ T cells and CD19⁺ B cells, no significant differences could be observed between both antibodies. Thus, although targeting different epitopes, vedolizumab and etrolizumab-s might similarly reduce adhesion of lymphocytes to MAdCAM-1 in intestinal vessels in vivo. However, this does not preclude differences in clinical efficacy or side effects, since α4β7 is also able to interact with VCAM-1 and fibronectin and the antibodies might differ in their interference with these interactions. Moreover, as etrolizumab is an anti-β7 antibody, it also impedes binding of αEβ7 to epithelial E-Cadherin. As suggested by a recent study with humanized mice, this might result in additional reduction of colonic lymphocyte numbers. Moreover, additional positive or negative effects due to modulation of αE (CD103)-expressing dendritic cells cannot be ruled out and αEβ7 is not gut specific, which might result in a different safety profile compared with vedolizumab. Therefore, we have been able to answer an important question by comparing vedolizumab and etrolizumab in dynamic adhesion assays but further translational and clinical research is needed to address these remaining points.

In contrast to T cells, not much is known about the gut homing mechanisms of other leukocyte subsets. It is known that B cells are increased in the intestinal mucosa of IBD patients suggesting an induction of homing in the context of intestinal inflammation and murine studies proposed that α4β7 plays an important role for B cell gut homing. We demonstrate that B cells may firmly adhere to MAdCAM-1, VCAM-1 and to a lesser extent also ICAM-1 suggesting that B lymphocytes may use all three pathways to infiltrate the gut.

Granulocytes are the first cells recruited to sites of inflammation and have considerable pathogenetic potential. In CD, lower homing capacity to the intestine has been reported and linked to defective chemoattractant signaling. However, while neutrophil granulocytes are generally believed to home dependent on αMβ2 and αLβ2 interacting with ICAM-1, the precise mechanisms of gut homing have been poorly studied so far. Our data confirm that granulocytes barely adhere to MAdCAM-1 and VCAM-1, but avidly adhere to ICAM-1, an effect that might even be underestimated since, in contrast to α4 integrins, β2 integrins have no additional role in rolling potentially making adhesion more difficult. This is consistent with the notion that gut homing of neutrophils is predominantly mediated by αMβ2 and αLβ2 integrins, which is in line with a study reporting reduced severity of experimental colitis in anti-αMβ2-treated rats going along with reduced intestinal granulocyte infiltration. Similarly, neutrophil influx in αL- and β2-deficient mice submitted to dextran sodium sulfate (DSS) colitis was reduced and colitis was less pronounced. Consistently, studies with anti-ICAM-1 antibodies or genetic disruption of ICAM-1 led to disease protection. Translation to human IBD showed no convincing success so far since alicaforsen, an antisense inhibitor of ICAM-1 failed to meet predefined primary endpoints in clinical trials, whereas efficacy could be demonstrated in post-hoc analyses of study subgroups. This might potentially arise from a dual role of ICAM-1-binding β2-integrins since αM-deficient mice were reported to develop more severe DSS colitis than wildtype control animals.

Using flow cytometric data on the expression of integrin expression on lymphocytes and granulocytes, we also correlated integrin expression and function, i.e. dynamic adhesion. On the whole, the picture emerged that both in innate immune cells and regarding integrins predominantly expressed by innate cells, function seems to be directly related to expression. On the contrary, adaptive immune cells and concerning α4β7 and α4β1, integrins considered predominantly important for the adaptive immune system, no clear association could be demonstrated. As mentioned above, this most probably results from further regulatory mechanisms affecting integrin-addressin interaction. Moreover, we could show that individual expression of α4β7 on CD4⁺ T cells is negatively related to adhesion to MAdCAM-1. On the first view, this is surprising, but might be a consequence of inter-individual differences of the importance of the α4β7-MAdCAM-1 pathway for gut homing. In this concept, an important role of α4β7 could result in high levels of adhesion to MAdCAM-1 accompanied by reduction of α4β7-expressing T cells in the peripheral blood due to preferential gut homing. This is well matching with data from a recent study from our lab showing that low baseline α4β7 expression before initiation of vedolizumab treatment might be associated with better clinical outcome than high baseline expression.

Since these results were consistent with the view that dynamic adhesion is partly but not fully related to integrin expression, direct assessment of integrin function might be more precise to estimate the potential clinical benefit of anti-adhesion therapies. Thus, in a pilot investigation, we analyzed dynamic adhesion of CD4⁺ T cells to MAdCAM-1 prior to initiation of vedolizumab treatment in two UC patients and subsequently in a cohort of 18 IBD patients. When patients were stratified according to response to vedolizumab, adhesion of untreated cells and the difference in adhesion of treated and non-treated cells was higher in patients that showed a response. Moreover, we were able to identify preliminary thresholds for this prediction by ROC analysis. Due to the liminted number of patients included, these preliminary findings remain should be interpreted cautiously, but, still, they support the notion that functional investigations with dynamic adhesion assays might provide useful information for clinical decisions. Prospective clinical studies with larger patient numbers will be needed to confirm these findings. However, if it should be possible to verify this hypothesis in such future studies, clinical implementation of the concept could be easier compared with other approaches reported for anti-TNF-a and etrolizumab therapy as these require the analysis of intestinal tissue.

Another frequent problem with biological therapies is a secondary loss of response during treatment. In this context, it is also imaginable that the dynamic adhesion technique could be used to demonstrate a concomitant re-occurence of adhesion to MAdCAM-1 or increased adhesion to other addressins under therapies such as vedolizumab. Moreover, it might be possible to evaluate in vitro, whether a dose escalation could overcome a loss of response and re-establish reduced addressin adhesion under anti-adhesion therapy.

Taken together, we report a useful technique for the real-time functional analysis of dynamic integrin-mediated cell adhesion to addressins with broad applicability in translational IBD research. Our data give novel insights into gut homing mechanisms and suggest comparable ability of vedolizumab and etrolizumab to disrupt of α4β7-dependent adhesion to MAdCAM-1, while promoting the idea that dynamic adhesion could be used to predict response to anti-adhesion therapy.

### References

Feagan BG, Rutgeerts P, Sands BE, et al. Vedolizumab as induction and maintenance therapy for ulcerative colitis. N. Engl. J. Med. 2013;369:699-710.
Feagan BG, Sandborn WJ, D'Haens G, et al. Randomised clinical trial: vercirnon, an oral CCR9 antagonist, vs. placebo as induction therapy in active Crohn's disease. Aliment Pharmacol Ther. 2015;42(10):1170-81.
Fischer A, Zundler S, Atreya R, et al. Differential effects of α4β7 and GPR15 on homing of effector and regulatory T cells from patients with UC to the inflamed gut in vivo. Gut. 2016;65:1642-1664
Leppkes M, Maueröder C, Hirth S, et al. Externalized decondensed neutrophil chromatin occludes pancreatic ducts and drives pancreatitis. Nat. Commun. 2016;7]
Lobatón T, Vermeire S, Van Assche G, et al. Review article: anti-adhesion therapies for inflammatory bowel disease. Aliment. Pharmacol. Ther. 2014;39:579-594.
Mayer L, Sandborn WJ, Stepanov Y, Anti-IP-10 antibody (BMS-936557) for ulcerative colitis: a phase II randomised study. Gut. 2014 Mar;63:442-50.
Pan WJ, Köck K, Rees WA, et al. Clinical pharmacology of AMG 181, a gut-specific human anti-α4β7 monoclonal antibody, for treating inflammatory bowel diseases. Br J Clin Pharmacol. 2014; 78:1315-33.
Sandborn WJ, Feagan BG, Rutgeerts P, et al. Vedolizumab as induction and maintenance therapy for Crohn's disease. N. Engl. J. Med. 2013;369:711-721.
Targan SR, Feagan BG, Fedorak RN, Natalizumab for the treatment of active Crohn's disease: results of the ENCORE Trial. Gastroenterology. 2007 May;132:1672-83.
Tidswell M, Pachynski R, Wu SW, et al. Structure-function analysis of the integrin beta 7 subunit: identification of domains involved in adhesion to MAdCAM-1. J. Immunol. 1997;159:1497-1505.
Vermeire S, O'Byrne S, Keir M, et al. Etrolizumab as induction therapy for ulcerative colitis: a randomised, controlled, phase 2 trial. Lancet 2014;384:309-318.
Vermeire S, Sandborn WJ, Danese S, et al. Anti-MAdCAM antibody (PF-00547659) for ulcerative colitis (TURANDOT): a phase 2, randomised, double-blind, placebo-controlled trial. Lancet 2017; 390:135-144.
Yoshimura N, Watanabe M, Motoya S, et al. Safety and Efficacy of AJM300, an Oral Antagonist of α4 Integrin, in Induction Therapy for Patients With Active Ulcerative Colitis. Gastroenterology. 2015; 149:1775-1783.e2.
Zundler S, Becker E, Weidinger C, et al. Anti-Adhesion Therapies in Inflammatory Bowel Disease-Molecular and Clinical Aspects. Front. Immunol. 2017;8:891.
Zundler S, Schillinger D, Fischer A, et al. Blockade of αEβ7 integrin suppresses accumulation of CD8+ and Th9 lymphocytes from patients with IBD in the inflamed gut in vivo. Gut. 2017;66:1936-1948

## Claims

1. Method for determining whether a patient with inflammatory bowel disease (IBD) will be responsive to an inhibitor of the interaction of endothelial adhesion molecules with gut homing receptors comprising the following steps:
(i) Moving a composition comprising cells of the patient along a surface coated with an endothelial adhesion molecule;
(ii) Detecting the gut homing receptor mediated adhesion of the cells to the surface coated with the endothelial adhesion molecule;
(iii) Predicting whether the patient with IBD will be responsive to the inhibitor of the interaction of endothelial adhesion molecules with gut homing receptors based on the result of step (ii).

2. Method according to claim 1, wherein in step (i) the composition comprising cells is moved through an elongated structure wherein the surface of the inner side of the elongated structure is coated with an endothelial adhesion molecule.

3. Method according to claim 2, wherein the elongated structure is a capillary.

4. Method according to any one of the preceding claims, wherein in step (ii) the adhesion of untreated cells to the coated surface is measured.

5. Method according to claim 4, wherein the adhesion of the untreated cells above a predetermined threshold is indicative for a response of the patient with IBD to the inhibitor of the interaction of endothelial adhesion molecules with gut homing receptors.

6. Method according to claims 1 to 3, wherein in step (ii) the surface adhesion of cells treated with the inhibitor is compared to the surface adhesion of control cells.

7. Method according to claim 6, wherein the decreased surface adhesion of the treated cells in comparison to the surface adhesion of the control cell is indicative for the efficacy of the inhibitor.

8. Method according to any one of the preceding claims, wherein the endothelial adhesion molecule is MAdCAM-1, ICAM-1 or VCAM-1, preferably wherein the endothelial adhesion molecule is MAdCAM-1 and wherein the gut homing receptor is integrin α4β7.

9. Method according to any one of the proceeding claims, wherein the cells are leukocytes, preferably CD4+ T cells or CD 8+ T cells.

10. Method according to any one of the preceding claims, wherein the inhibitor of the interaction of endothelial adhesion molecules with gut homing receptors is an antibody targeting endothelial adhesion molecules or an antibody targeting gut homing receptors.

11. Method according to any one of the preceding claims, wherein the inhibitor of the interaction of endothelial adhesion molecules with gut homing receptors is selected from the group consisting of vedolizumab, natalizumab and etrolizumab and SHP647.

12. Method according to any one of the preceding claims, wherein the cells are fluorescently labeled and wherein the detection in step (ii) is fluorescence detection, preferably performed by time-lapse microscopy.

13. Method for determining whether a candidate compound is suitable for the treatment of IBD comprising the following steps:
(i) Moving a composition comprising cells along a surface coated with an endothelial adhesion molecule;
(ii) Detecting gut homing receptor mediated adhesion of the cells to the surface coated with the endothelial adhesion molecule, wherein the surface adhesion of cells treated with the inhibitor is compared to control cells;
(iii) Predicting whether a candidate compound is suitable for the treatment of IBD based on the result of step (ii).

14. Kit comprising:
- an elongated structure coated at its inner surface with an endothelial adhesion molecule,
- instructions for use,
- optionally an adhesion buffer,
- optionally a labeling compound.

15. Device comprising:
- means for accommodating a capillary,
- means capable of moving the composition comprising cells through the capillary,
- optionally a reservoir capable of accommodating a composition comprising cells,
- optionally a reservoir for incubation of a composition comprising cells with the inhibitor of the interaction of a gut homing receptor with an endothelial adhesion molecule,
- optionally a detector capable of detecting the surface adhesion of the cells in the capillary,
- optionally means for determining the number of adhering cells,
- optionally means for comparing the determined value with a specific threshold value and/or for calculating the probability of response based on the determined value,
- optionally a display indicating the result of the measurement, the comparison and/or the calculation,
- optionally means for connecting a coated capillary with the reservoir.
